# EUROPEAN PATENT APPLICATION

(11) **EP 2 641 544 A2**
(43) Date of publication of application: **25.09.2013**
(21) Application number: 11840821.0
(22) Date of filing: 14.11.2011
(51) Int. Cl.: A61B 8/14, G06T 5/00

(54) **COLOR FLOW IMAGING METHOD, AND ULTRASONIC DEVICE THEREFOR**

(30) Priority: 16.11.2010 KR 20100113848
(71) Applicant: Alpinion Medical Systems Co., Ltd., Hwaseong-si, Gyeonggi-do 445-380 (KR)
(72) Inventor: EOM, Minyoung, Seoul 100-440 (KR)
(74) Representative: Thorniley, Peter
(86) International application number: PCT/KR2011/008669
(87) International publication number: WO 2012/067391

(57) **Abstract**

A color flow imaging method and an ultrasonic imaging system are provided. The ultrasonic imaging system includes: a transceiver unit configured to transmit an ultrasonic wave signal for acquisition of a C-mode image to a subject and receive an ultrasonic wave echo signal reflected from the subject; a storage unit configured to store the received echo signal; a control unit configured to output an interpolated signal by interpolating ensembles of at least one scan line for acquisition of the C-mode image identified based on the received echo signal; and an image processor configured to form the C-mode image based on the interpolated signal and to output the C-mode image on a display unit.

## Description

### [Cross-Reference to Related Application]

The present application is the US national phase of International Patent Application No. PCT/KR2011/008669, filed November 14, 2011, which claims priority to Korean Patent Application No. 10-2010-0113848, filed on November 16, 2010. The disclosures of the above-listed applications are hereby incorporated by reference in their entirety.

### [Technical Field]

The present disclosure in some embodiments relates to a color flow imaging method and an ultrasonic imaging system. More particularly, the present disclosure relates to a color flow imaging method for acquiring more accurate blood flow information at the same frame rate by using a signal with ensembles interpolated, and for obtaining approximately twice the frame rate at the same pulse repetition frequency (PRF), and to an ultrasonic imaging system therefor.

### [Background]

The statements in this section merely provide background information related to the present disclosure and may not constitute prior art.

In general, ultrasonic imaging systems are important diagnostic systems for multi-industrial applications. In particular, thanks to the noninvasive and nondestructive characteristics to a subject, the ultrasonic imaging systems are used all over the medical field. The ultrasonic imaging systems typically provide a BC-mode image showing the state of blood flows or movements of the subject with the use of Doppler effect. The BC-mode is a mode for providing both of a gray scale B-mode image and a color flow image (that is, C-mode image) showing the state of blood flows or movements of the subject, and may provide anatomic information along with information about the blood flows and movements of the subject.

In order to accurately estimate blood flow information, the ultrasonic imaging systems are required to obtain a high frame rate by transmitting/receiving an ultrasonic wave signal many times. In particular, in the case of echocardiography, a high frame rate is required. Thus, a method of increasing a PRF is used instead of a method of reducing the number of repetitions of transmission/reception of an ultrasonic wave signal with respect to a subject. However, the number of repetitions of transmission/reception of the ultrasonic wave signal with respect to the subject is reduced in order to maintain a high frame rate. As a result, the performance of a filter used for removing clutter components may be degraded.

### [Disclosure]

### [Technical Problem]

Therefore, the present disclosure has been made in an effort to effectively resolving the above-described limitations and provides a color flow imaging method for estimating accurate blood flow information by using a signal with interpolated ensembles, and an ultrasonic imaging system therefor.

### [Summary]

An embodiment of the present disclosure provides an ultrasonic imaging system, including a transceiver unit, a storage unit, a control unit and an image processor. The transceiver unit is configured to transmit an ultrasonic wave signal for acquisition of a C-mode image to a subject and receive an ultrasonic wave echo signal reflected from the subject. The storage unit is configured to store the received echo signal. The control unit is configured to output an interpolated signal with one or more ensembles interpolated with respect to a scan line for acquisition of the C-mode image identified based on the received echo signal. And the image processor is configured to form the C-mode image based on the interpolated signal and to output the C-mode image on a display unit.

Another embodiment of the present disclosure provides an ultrasonic imaging system, including a transceiver unit, a storage unit, a control unit and an image processor. The transceiver unit is configured to transmit an ultrasonic wave signal for acquisition of a C-mode image to a subject and receive an ultrasonic wave echo signal reflected from the subject to form a received echo signal. The storage unit is configured to store the received echo signal. The control unit is configured to output an interpolated signal in which zero insertion is performed on one or more ensembles of a scan line for acquisition of the C-mode image identified based on the received echo signal. And the image processor is configured to form the C-mode image based on the interpolated signal and output the C-mode image on a display unit.

Yet another embodiment of the present disclosure provides an ultrasonic imaging system, including a transceiver unit, a storage unit, a control unit and an image processor. The transceiver unit is configured to transmit an ultrasonic wave signal for acquisition of a C-mode image to a subject and receive an ultrasonic wave echo signal reflected from the subject to form a received echo signal. The storage unit is configured to store the received echo signal. The control unit is configured to repeat one or more ensembles one cycle in time domain by applying a periodic extension to the ensembles of a scan line for acquisition of the C-mode image identified based on the received echo signal and output an interpolated signal. And the image processor is configured to form the C-mode image based on the interpolated signal and output the C-mode image on a display unit.

Yet another embodiment of the present disclosure provides an ultrasonic imaging system, including a transceiver unit, a storage unit, a control unit and an image processor. The transceiver unit is configured to transmit an ultrasonic wave signal for acquisition of a C-mode image to a subject and receive an ultrasonic wave echo signal reflected from the subject to form a received echo signal. The storage unit is configured to store the received echo signal. The control unit is configured to output an interpolated signal in which a symmetric extension is applied to one or more ensembles of a scan line for acquisition of the C-mode image identified based on the received echo signal. And the image processor is configured to form the C-mode image based on the interpolated signal and output the C-mode image on a display unit.

Yet another embodiment of the present disclosure provides an ultrasonic imaging system, including a transceiver unit, a storage unit, a control unit and an image processor. The transceiver unit is configured to transmit an ultrasonic wave signal for acquisition of a C-mode image to a subject and receive an ultrasonic wave echo signal reflected from the subject to form a received echo signal. The storage unit is configured to store the received echo signal. The control unit is configured to output an interpolated signal in which an anti-symmetric extension is applied to one or more ensembles of a scan line for acquisition of the C-mode image identified based on the received echo signal. And the image processor is configured to form the C-mode image based on the interpolated signal and output the C-mode image on a display unit.

Yet another embodiment of the present disclosure provides a color flow imaging method, including transmitting an ultrasonic wave signal for acquisition of a C-mode image to a subject and receiving an ultrasonic wave echo signal reflected from the subject to form a received echo signal; outputting an interpolated signal with one or more ensembles interpolated with respect to a scan line for acquisition of the C-mode image identified based on the received echo signal; and forming the C-mode image based on the interpolated signal and outputting the C-mode image on a display unit.

Yet another embodiment of the present disclosure provides a color flow imaging method, including transmitting an ultrasonic wave signal for acquisition of a C-mode image to a subject and receiving an ultrasonic wave echo signal reflected from the subject to form a received echo signal; outputting an interpolated signal in which a zero insertion is performed on one or more ensembles of a scan line for acquisition of the C-mode image identified based on the received echo signal; and forming the C-mode image based on the interpolated signal and outputting the C-mode image on a display unit.

Yet another embodiment of the present disclosure provides a color flow imaging method, including transmitting an ultrasonic wave signal for acquisition of a C-mode image to a subject and receiving an ultrasonic wave echo signal reflected from the subject to form a received echo signal; repeating one or more ensembles one cycle in time domain by applying a periodic extension to the ensembles of a scan line for acquisition of the C-mode image identified based on the received echo signal and outputting an interpolated signal; and forming the C-mode image based on the interpolated signal and outputting the C-mode image on a display unit.

Yet another embodiment of the present disclosure provides a color flow imaging method, including transmitting an ultrasonic wave signal for acquisition of a C-mode image to a subject and receiving an ultrasonic wave echo signal reflected from the subject to form a received echo signal; outputting an interpolated signal in which a symmetric extension is applied to one or more ensembles of a scan line for acquisition of the C-mode image identified based on the received echo signal; and forming the C-mode image based on the interpolated signal and outputting the C-mode image on a display unit.

Yet another embodiment of the present disclosure provides a color flow imaging method, including transmitting an ultrasonic wave signal for acquisition of a C-mode image to a subject and receiving an ultrasonic wave echo signal reflected from the subject to form a received echo signal; outputting an interpolated signal in which an anti-symmetric extension is applied to one or more ensembles of a scan line for acquisition of the C-mode image identified based on the received echo signal; and forming the C-mode image based on the interpolated signal and outputting the C-mode image on a display unit.

### [Advantageous Effects]

According to the present disclosure as described above, it is possible to acquire more accurate blood flow information at the same frame rate by using a signal with ensembles interpolated, and obtain about twice the frame rate at the same PRF. That is, the ultrasonic imaging system generates 2n ensembles by transmitting an ultrasonic wave signal to a subject n times and interpolating n ensembles identified through corresponding ultrasonic wave echo signals. Therefore, more accurate blood flow information can be acquired at the same PRF.

In addition, according to the present disclosure as described above, the ultrasonic imaging system generates n ensembles by transmitting an ultrasonic wave signal to a subject n/2 times and interpolating n/2 ensembles identified after corresponding ultrasonic wave signals are received. Therefore, it is possible to obtain the same frame rate as that of the case when n ensembles are generated and an ultrasonic wave signal is transmitted n times. That is, the ultrasonic imaging system can obtain approximately twice the frame rate while guaranteeing the same performance as that of the case where n ensembles are generated and an ultrasonic wave signal is transmitted n times.

### [Description of Drawings]

FIG. 1 is a schematic block diagram of an ultrasonic imaging system according to at least one embodiment of the present disclosure;

FIG. 2 is a flowchart of a color flow imaging method according to at least one embodiment of the present disclosure;

FIGs. 3A and 3B are exemplary diagrams of a method for interpolating an ensemble by performing zero insertion according to at least one embodiment of the present disclosure;

FIG. 4 is an exemplary diagram of a method for interpolating an ensemble by applying periodic extension according to at least one embodiment of the present disclosure;

FIGs. 5A and 5B are exemplary diagrams of a method for interpolating an ensemble by applying symmetric extension according to at least one embodiment of the present disclosure; and

FIGs. 6A and 6B are exemplary diagrams of a method for interpolating an ensemble by applying anti-symmetric extension according to at least one embodiment of the present disclosure.

### <Reference Signs List>

| | | | |
|---|---|---|---|
| 100: | ultrasonic imaging system | 110: | user input unit |
| 120: | transceiver unit | 130: | storage unit |
| 140: | control unit | 150: | signal processor |
| 160: | image processor | 170: | display unit |

### [Detailed Description]

Hereinafter, at least one embodiment of the present disclosure will be described in detail with reference to the accompanying drawings. In the following description, like reference numerals designate like elements although the elements are shown in different drawings. Further, in the following description of the at least one embodiment, a detailed description of known functions and configurations incorporated herein will be omitted for the purpose of clarity and for brevity.

Additionally, in describing the components of the present disclosure, terms like first, second, A, B, (a), and (b) are used. These are solely for the purpose of differentiating one component from another, and one of ordinary skill would understand the terms are not to imply or suggest the substances, order or sequence of the components. If a component is described as 'connected', 'coupled', or 'linked' to another component, one of ordinary skill in the art would understand the components are not necessarily directly 'connected', 'coupled', or 'linked' but also are indirectly 'connected', 'coupled', or 'linked' via a third component.

In the present disclosure, a B-mode is a gray scale image mode showing the state of blood flows or movements of the subject, and a C-mode is a color flow image mode showing the state of blood flows or movements of the subject. That is, the B-mode and the C-mode are image modes that can provide anatomic information along with information about the blood flows and movements of the subject. Meanwhile, an ultrasonic imaging system 100 according to at least one embodiment of the disclosure simultaneously provides the B-mode image and the color flow image (that is, the C-mode image). More particularly, the ultrasonic imaging system 100 provides a BC-mode image that is a combination of the B-mode image and the C-mode image. However, for the sake of convenience, the following description will focus on the ultrasonic imaging system 100 configured to form the C-mode image.

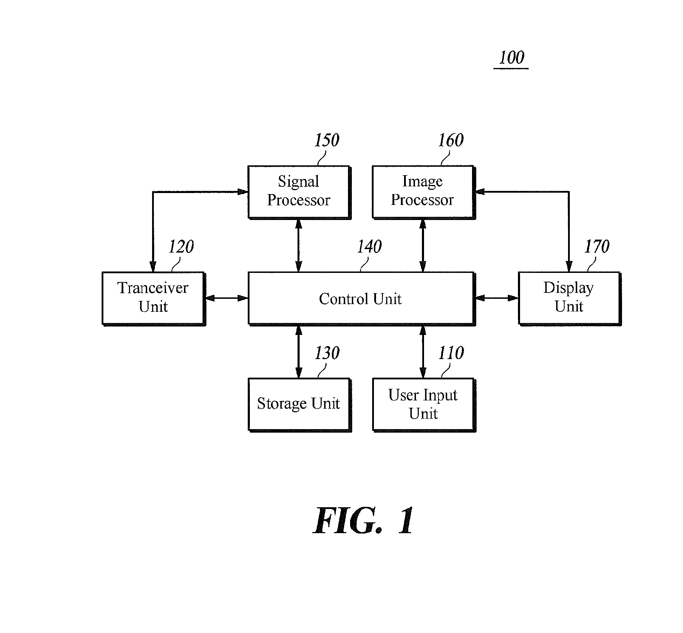

FIG. 1 is a schematic block diagram of an ultrasonic imaging system according to at least one embodiment of the present disclosure.

The ultrasonic imaging system 100 according to at least one embodiment of the present disclosure includes a user input unit 110, a transceiver unit 120, a storage unit 130, a control unit 140, a signal processor 150, an image processor 160, and a display unit 170. Although the ultrasonic imaging system 100 according to at least one embodiment of the present disclosure has been described as including only the user input unit 110, the transceiver unit 120, the storage unit 130, the control unit 140, the signal processor 150, the image processor 160, and the display unit 170, this is merely for exemplary description about the technical spirit of at least one embodiment of the present disclosure. Therefore, those skilled in the art will appreciate that various modifications, additions and substitutions for the components included in the ultrasonic imaging system 100 are possible, without departing from the essential characteristics of at least one embodiment of the present disclosure.

The user input unit 110 receives a user instruction generated by a user's manipulation or input. The user input unit 110 receives Region of Interest (ROI) setting information. The ROI includes a color box, and the ROI setting information includes information about the location and size of the ROI. In this case, the user instruction may be a selection instruction for selecting an image mode of the ultrasonic imaging system 100, a setting instruction for setting the color box in a B-mode image, and the like. Meanwhile, the user instruction includes a setting instruction for setting ensembles in a C-mode. In other words, the ensembles represent the number of repetitions of transmission/reception of an ultrasonic wave signal with respect to each scan line in order to form a C-mode image, and refers to a type of packet number. The ensembles may be previously set to the ultrasonic imaging system 100 or may be manually set in response to input of a setting instruction from the user.

In order to acquire the C-mode image, the transceiver unit 120 is configured to form a received echo signal by transmitting an ultrasonic wave signal to the subject and then receiving an ultrasonic wave echo signal reflected from the subject. Specifically, the transceiver unit 120 forms the received echo signal by transmitting the ultrasonic wave signal to the subject and receiving the ultrasonic wave echo signal reflected from the subject, based on a control signal received from the control unit 140. In addition, the transceiver unit 120 forms the received echo signal by transmitting/receiving the ultrasonic wave signal at a PRF with respect to the ROI, based on the control signal received from the control unit 140. In this case, the received echo signal includes a Doppler signal and a clutter signal. The Doppler signal is a signal obtained when the ultrasonic wave signal from the transceiver unit 120 is reflected by the blood flow. The Doppler signal is relatively high in frequency but relatively weak in intensity. The clutter signal is a signal obtained when the ultrasonic wave signal from the transceiver unit 120 is reflected from a cardiac wall or cardiac plate. The clutter signal is relatively low in frequency but relatively high in intensity.

On the other hand, the transceiver unit 120 includes a probe (not shown) configured to transmit/receive the ultrasonic wave signal, and a beamformer (not shown) configured to perform the transmission focusing and reception focusing of the ultrasonic wave signal. The probe includes a plurality of one-dimensional (1D) or two-dimensional (2D) array transducers. The probe transmits a focused ultrasonic wave beam to a subject (not shown) along a transmission scan line by appropriately delaying an input time of pulses input to the respective transducers. Meanwhile, the ultrasonic wave echo signals reflected from the subject are input to the respective transducers with different reception times, and the respective transducers output the input ultrasonic wave echo signals to the beamformer. The beamformer focuses the ultrasonic wave signals on a specific location by adjusting the driving timings of the respective transducers within the probe when the probe transmits the ultrasonic wave signal, and focuses the ultrasonic wave echo signals by adding a delay time to the respective ultrasonic wave echo signals of the probe, taking into consideration the fact that the ultrasonic wave echo signals reflected from the subject reach the respective transducers of the probe at different times.

The storage unit 130 stores the received echo signal formed by the transceiver unit 120. In addition, the storage unit 130 stores a plurality of pieces of cutoff frequency information for eliminating the clutter signal from the received echo signal.

The control unit 140 is a controller configured to control the overall operation of the ultrasonic imaging system 100. The control unit 140 according to at least one embodiment of the present disclosure is configured to output an interpolated signal with ensembles interpolated with respect to a scan line for acquisition of the C-mode image identified based on the received echo signal formed by the transceiver unit 120. The control unit 140 controls the transceiver unit 120 such that the ensembles by the number of n are reduced by decreasing the number of repetitions of transmission/reception of the ultrasonic wave signal with respect to the scan line for acquisition of the C-mode image by a predetermined number of times, and enables the transceiver unit 120 to form the received echo signal corresponding to the reduced ultrasonic wave signal. The control unit 140 controls the transceiver unit 120 such that the ensembles are reduced by half by halving the number of repetitions of transmission/reception of the ultrasonic wave signal with respect to the scan line for acquisition of the C-mode image. Meanwhile, in a case where the number of repetitions of transmission/reception of the ultrasonic wave signal with respect to the scan line for acquisition of the C-mode image is set by the user input unit 110, the control unit 140 controls the transceiver unit 120 such that the transceiver unit 120 forms the received echo signal corresponding to the set ultrasonic wave signal.

The control unit 140 outputs the interpolated signal by performing zero insertion on the reduced ensembles or the set ensembles. Specifically, the control unit 140 outputs the interpolated signal in which zero insertion is performed between the respective ensembles. The control unit 140 repeats the ensembles one cycle in time domain by applying periodic extension to the reduced ensembles or the set ensembles and then outputs the interpolated signal. On the other hand, if the control unit 140 repeats the ensembles one cycle by applying the periodic extension in a state where the ensembles have not been reduced and then outputs the interpolated signal, the performance can be improved about two times at a frame rate. The control unit 140 outputs the interpolated signal in which symmetric extension is applied to the reduced ensembles or the set ensembles. In this case, after making the reduced ensembles or the set ensembles symmetric, the control unit 140 repeats the symmetric ensembles one cycle in time domain. When symmetric extension is applied, the control unit 140 repeats the ensembles one cycle from the second value of the values of the symmetric ensembles. The control unit 140 outputs the interpolated signal in which anti-symmetric extension is applied to the reduced ensembles or the set ensembles. In this case, after making the reduced ensembles or the set ensembles anti-symmetric, the control unit 140 repeats the anti-symmetric ensembles one cycle in time domain. When anti-symmetric extension is applied, the control unit 140 performs zero insertion once between the reduced ensembles (or the set ensembles) and the anti-symmetric ensembles. That is, the control unit 140 reduces the number of repetitions of transmission/reception of the ultrasonic wave signal with respect to the scan line for acquisition of the C-mode image by a predetermined number of times by applying the zero insertion, periodic extension, symmetric extension and anti-symmetric extension to the reduced ensembles. In this case, the frame rate can be improved about two times while maintaining similar performance to that before the reduction.

On the other hand, when ROI setting information is input from the user input unit 110, the control unit 140 may control the transmission/reception of the ultrasonic wave signal by using the ROI setting information. In addition, the control unit 140 repeatedly performs the transmission/reception of the ultrasonic wave signal for acquisition of the B-mode image and the transmission/reception of the ultrasonic wave signal for acquisition of the C-mode image.

The signal processor 150 sets a plurality of filters having cutoff frequencies for eliminating a clutter signal to respective pixels within the ROI, and performs clutter filtering on the received echo signal from the transceiver unit 120. Meanwhile, the signal processor 150 may perform signal processing such as gain control in order for image optimization of the received echo signal from the transceiver unit 120. In addition, the signal processor 150 transmits the interpolated signal to the image processor 160 after low-pass filtering.

The image processor 160 is configured to form the C-mode image based on the interpolated signal and output the C-mode image on the display unit 170. Specifically, the image processor 160 forms the B-mode image or C-mode image by using the received echo signal formed by the signal processor 150. In addition, the image processor 160 outputs the formed B-mode image or C-mode image on the display unit 170.

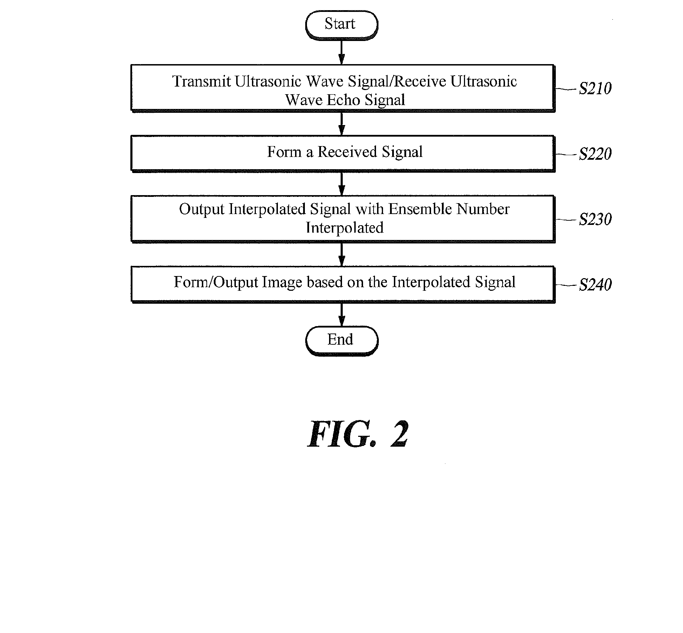

FIG. 2 is a flowchart of a color flow imaging method according to at least one embodiment of the present disclosure.

The ultrasonic imaging system 100 transmits an ultrasonic wave signal for acquisition of a C-mode image to a subject and receives an ultrasonic wave echo signal reflected from the subject by means of the transceiver unit 120 (S210), and forms a received echo signal corresponding to the ultrasonic wave echo signal (S220). The ultrasonic imaging system 100 outputs an interpolated signal with ensembles interpolated with respect to a scan line for acquisition of the C-mode image identified based on the received echo signal formed by the transceiver unit 120 (S230). In this case, the ultrasonic imaging system 100 controls the transceiver unit 120 such that the ensembles by the number of n are reduced by decreasing the number of repetitions of transmission/reception of the ultrasonic wave signal with respect to the scan line for acquisition of the C-mode image by a predetermined number of times, and enables the transceiver unit 120 to form the received echo signal corresponding to the reduced ultrasonic wave signal. That is, the ultrasonic imaging system 100 controls the transceiver unit 120 such that the ensembles are reduced by half by halving the number of repetitions of transmission/reception of the ultrasonic wave signal with respect to the scan line for acquisition of the C-mode image. Meanwhile, when the number of repetitions of transmission/reception of the ultrasonic wave signal with respect to the scan line for acquisition of the C-mode image is set by the user input unit 110, the ultrasonic imaging system 100 controls the transceiver unit 120 such that the transceiver unit 120 forms the received echo signal corresponding to the set ultrasonic wave signal.

The ultrasonic imaging system 100 performs zero insertion on the reduced ensembles or the set ensembles and then outputs the interpolated signal. The ultrasonic imaging system 100 repeats the ensembles one cycle in time domain by applying periodic extension to the reduced ensembles or the set ensembles, and then outputs the interpolated signal. The ultrasonic imaging system 100 outputs the interpolated signal in which symmetric extension is applied to the reduced ensembles or the set ensembles. In this case, after making the reduced ensembles or the set ensembles symmetric, the ultrasonic imaging system 100 repeats the symmetric ensembles one cycle in time domain. When symmetric extension is applied, the ultrasonic imaging system 100 repeats the ensembles one cycle from the second value of the values of the symmetric ensemble. The ultrasonic imaging system 100 outputs the interpolated signal in which anti-symmetric extension is applied to the reduced ensemble or the set ensemble. In this case, after making the reduced ensemble or the set ensemble anti-symmetric, the ultrasonic imaging system 100 repeats the anti-symmetric ensembles one cycle in time domain. When anti-symmetric extension is applied, the ultrasonic imaging system 100 performs zero insertion once between the reduced ensembles (or the set ensembles) and the anti-symmetric ensembles. The ultrasonic imaging system 100 forms the C-mode image based on the interpolated signal and outputs the C-mode image on the display unit 170 (S240).

Although steps S210 to S240 of FIG. 2 have been described as being sequentially performed, this is only exemplary description of the technical spirit of at least one embodiment of the present disclosure. Those skilled in the art will appreciate that various modifications, additions and substitutions are possible, without departing from the essential characteristics of at least one embodiment of the disclosure. For example, the order of steps shown in FIG. 2 may be changed, or one or more of steps 210 to 240 may be performed in parallel. Therefore, the present disclosure is not limited to the time-series order as shown in FIG. 2.

The color flow imaging method as described above and shown in FIG. 2 may be embodied as a program stored in a computer-readable recording medium. The computer-readable recording medium storing the program for realizing the color flow imaging method according to at least one embodiment of the present disclosure may be any data storage device that can store data which can be thereafter read by a computer system. Examples of the computer-readable recording medium include ROMs, RAMs, CD-ROMs, magnetic tapes, floppy disks, optical data storage devices, and carrier waves (such as data transmission through the Internet). The computer-readable recording medium may also be distributed over network coupled to computer systems so that computer-readable codes are stored and executed in a distributed fashion. In addition, functional programs, codes, and code segments for accomplishing at least one embodiment of the present disclosure may be easily construed by programmers skilled in the art to which the present disclosure pertains.

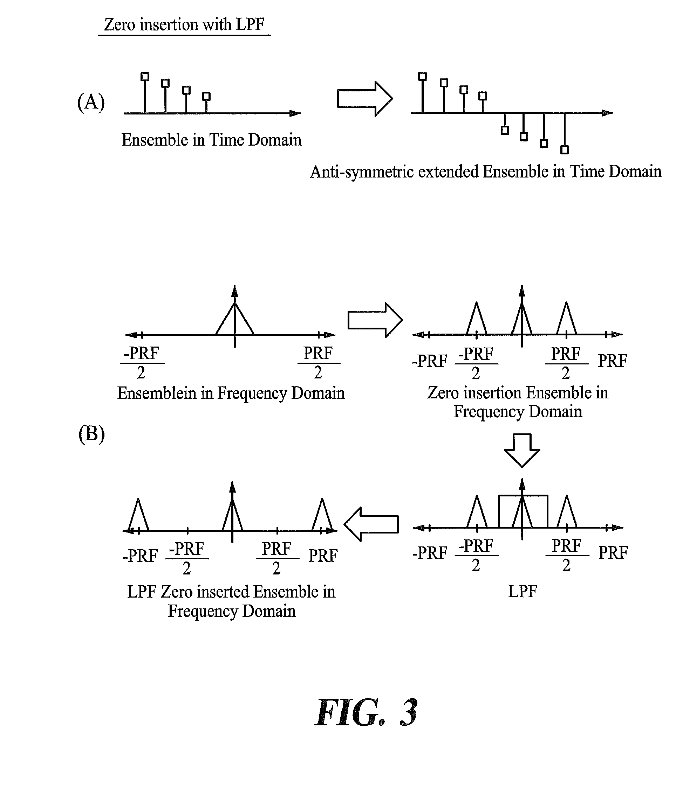

FIGs. 3A and 3B are exemplary diagrams of a method for interpolating an ensemble by performing zero insertion according to at least one embodiment of the present disclosure.

The ultrasonic imaging system 100 is configured to output the interpolated signal with the ensembles by the number of n interpolated with respect to the scan line for acquisition of the C-mode image identified based on the received echo signal formed by the transceiver unit 120. Specifically, the ultrasonic imaging system 100 controls the transceiver unit 120 such that the (n) ensembles is reduced by decreasing the number of repetitions of transmission/reception of the ultrasonic wave signal with respect to the scan line for acquisition of the C-mode image by a predetermined number of times, and enables the transceiver unit 120 to form the received echo signal corresponding to the reduced ultrasonic wave signal. The ultrasonic imaging system 100 may control the transceiver unit 120 such that the ensembles are reduced by half by halving the number of repetitions of transmission/reception of the ultrasonic wave signal with respect to the scan line for acquisition of the C-mode image, but the present disclosure is not limited thereto. When the number of repetitions of transmission/reception of the ultrasonic wave signal with respect to the scan line for acquisition of the C-mode image is set by the user input unit 110, the ultrasonic imaging system 100 controls the transceiver unit 120 such that the transceiver unit 120 forms the received echo signal corresponding to the set ultrasonic wave signal. As shown in FIG. 3A, the ultrasonic imaging system 100 is configured to output the interpolated signal in which zero insertion is performed on the (n) ensembles of the scan line for acquisition of the C-mode image identified based on the received echo signal formed by the transceiver unit 120. That is, the ultrasonic imaging system 100 performs zero insertion on the ensembles in time domain and then outputs the interpolated signal. As shown in FIG. 3B, the ultrasonic imaging system 100 performs zero insertion on the ensembles in frequency domain and then outputs the interpolated signal which has passed through a low pass filter (LPF). Specifically, zero insertion is performed in frequency domain with respect to ensembles between -PRF/2 and PRF/2 to generate the frequency domain of the ensembles at -PRF /2 and PRF/2. When the frequency domain passes through the LPF, the frequency domain is shown at -PRF and PRF.

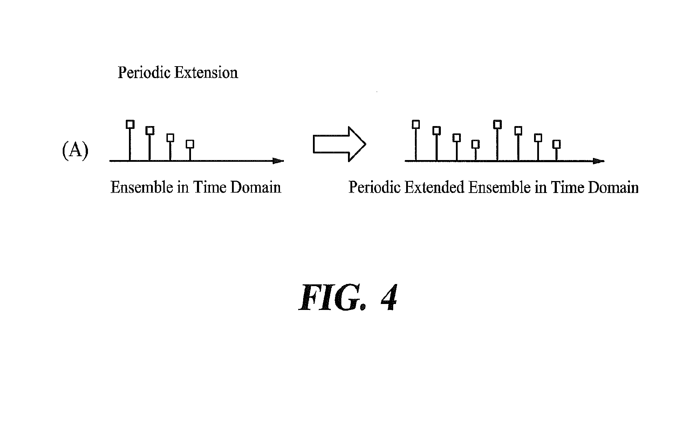

FIG. 4 is an exemplary diagram of a method for interpolating an ensemble by applying periodic extension according to at least one embodiment of the present disclosure.

The ultrasonic imaging system 100 repeats the ensembles one cycle in time domain by applying the periodic extension to the reduced ensembles or the set ensembles, and then outputs the interpolated signal. As shown in FIG. 4, the ultrasonic imaging system 100 is configured to output the interpolated signal in which periodic extension is performed on the (n) ensembles of the scan line for acquisition of the C-mode image identified based on the received echo signal formed by the transceiver unit 120. That is, the ultrasonic imaging system 100 repeats the ensembles one cycle in time domain and then outputs the interpolated signal.

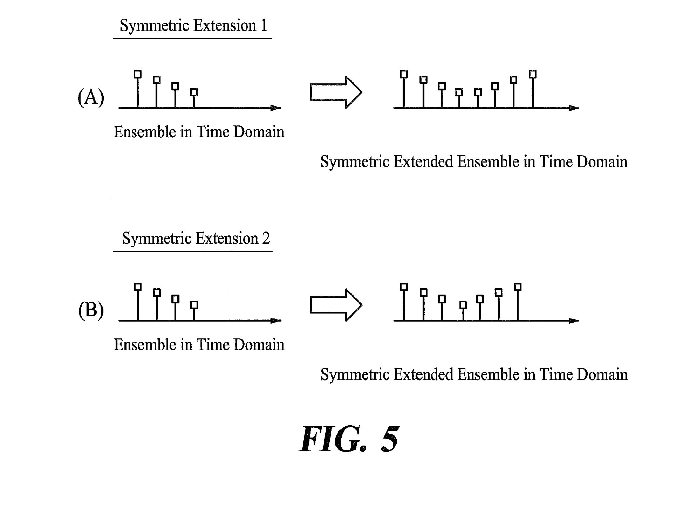

FIGs. 5A and 5B are exemplary diagrams of a method for interpolating an ensemble by applying symmetric extension according to at least one embodiment of the present disclosure.

The ultrasonic imaging system 100 outputs the interpolated signal in which symmetric extension is applied to the reduced ensembles or the set ensembles. In this case, after making the reduced ensembles or the set ensembles symmetric, the ultrasonic imaging system 100 repeats the symmetric ensembles one cycle in time domain. When symmetric extension is applied, the ultrasonic imaging system 100 repeats the ensembles one cycle from the second value of the values of the symmetric ensembles. As shown in FIG. 5A, the ultrasonic imaging system 100 is configured to output the interpolated signal in which symmetric extension is performed on the (n) ensembles of the scan line for acquisition of the C-mode image identified based on the received echo signal formed by the transceiver unit 120. That is, after making the ensembles symmetric, the ultrasonic imaging system 100 repeats the symmetric ensembles one cycle in time domain and then outputs the interpolated signal. As shown in FIG. 5B, the ultrasonic imaging system 100 is configured to output the interpolated signal in which the symmetric extension is performed on the (n) ensembles of the scan line for acquisition of the C-mode image identified based on the received echo signal formed by the transceiver unit 120. That is, when the symmetric extension is applied after making the ensembles symmetric, the ultrasonic imaging system 100 repeats the ensembles one cycle from the second value of the values of the symmetric ensembles and then outputs the interpolated signal.

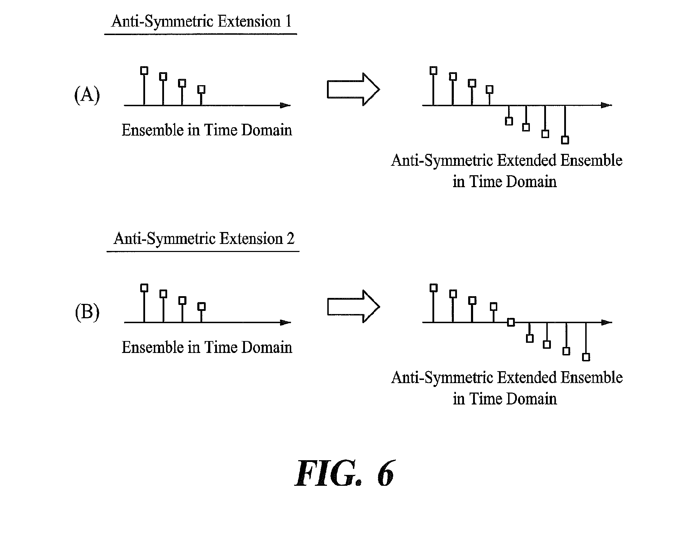

FIGs. 6A and 6B are exemplary diagrams of a method for interpolating an ensemble by applying anti-symmetric extension according to at least one embodiment of the present disclosure.

The ultrasonic imaging system 100 outputs the interpolated signal in which anti-symmetric extension is applied to the reduced ensembles or the set ensembles. In this case, after making the reduced ensembles or the set ensembles anti-symmetric, the ultrasonic imaging system 100 repeats the anti-symmetric ensembles one cycle in time domain. When anti-symmetric extension is applied, the ultrasonic imaging system 100 performs zero insertion once between the reduced ensembles (or the set ensembles) and the anti-symmetric ensembles. As shown in FIG. 6A, the ultrasonic imaging system 100 is configured to output the interpolated signal in which anti-symmetric extension is performed on the (n) ensembles of the scan line for acquisition of the C-mode image identified based on the received echo signal formed by the transceiver unit 120. That is, after making the ensembles anti-symmetric, the ultrasonic imaging system 100 repeats the anti-symmetric ensembles one cycle in time domain and then outputs the interpolated signal. As shown in FIG. 6B, the ultrasonic imaging system 100 is configured to output the interpolated signal in which the anti-symmetric extension is performed on the (n) ensembles of the scan line for acquisition of the C-mode image identified based on the received echo signal formed by the transceiver unit 120. That is, when the anti-symmetric extension is applied after making the ensembles anti-symmetric, the ultrasonic imaging system 100 performs zero insertion once between the ensemble and the anti-symmetric ensemble.

In the description above, although all of the components of the embodiments of the present disclosure may have been explained as assembled or operatively connected as a unit, one of ordinary skill would understand the present disclosure is not limited to such embodiments. Rather, within some embodiments of the present disclosure, the respective components are selectively and operatively combined in any number of ways. Every one of the components are capable of being implemented alone in hardware or combined in part or as a whole and implemented in a computer program having program modules residing in computer readable media and causing a processor or microprocessor to execute functions of the hardware equivalents. Codes or code segments to constitute such a program are understood by a person skilled in the art. The computer program is stored in a non-transitory computer readable media, which in operation realizes the embodiments of the present disclosure. The computer readable media includes magnetic recording media, optical recording media or carrier wave media, in some embodiments.

In addition, one of ordinary skill would understand terms like 'include', 'comprise', and 'have' to be interpreted in default as inclusive or open rather than exclusive or closed unless expressly defined to the contrary. All the terms that are technical, scientific or otherwise agree with the meanings as understood by a person skilled in the art unless defined to the contrary. One of ordinary skill would understand common terms as found in dictionaries are interpreted in the context of the related technical writings not too ideally or impractically unless the present disclosure expressly defines them so.

Although exemplary embodiments of the present disclosure have been described for illustrative purposes, those skilled in the art will appreciate that various modifications, additions and substitutions are possible, without departing from the essential characteristics of the disclosure. Therefore, exemplary embodiments of the present disclosure have been described for the sake of brevity and clarity. Accordingly, one of ordinary skill would understand the scope of the disclosure is not limited by the explicitly described above embodiments but by the claims and equivalents thereof.

### CROSS-REFERENCE TO RELATED APPLICATION

If applicable, this application claims priority under 35 U.S.C §119(a) of Patent Application No. 10-2010-0113848, filed on November 16, 2010 in Korea, the entire content of which is incorporated herein by reference. In addition, this non-provisional application claims priority in countries, other than the U.S., with the same reason based on the Korean Patent Application, the entire content of which is hereby incorporated by reference.

## Claims

1. An ultrasonic imaging system, comprising:
a transceiver unit configured to transmit an ultrasonic wave signal for acquisition of a C-mode image to a subject and receive an ultrasonic wave echo signal reflected from the subject;
a storage unit configured to store the received echo signal;
a control unit configured to output an interpolated signal by interpolating ensembles of at least one scan line for acquisition of the C-mode image identified based on the received echo signal; and
an image processor configured to form the C-mode image based on the interpolated signal and to output the C-mode image on a display unit.

2. The ultrasonic imaging system of claim 1, wherein the control unit controls the transceiver unit such that the number of the ensembles is reduced by decreasing the number of repetitions of transmission/reception of the ultrasonic wave signal with respect to the scan line for acquisition of the C-mode image by a predetermined number of times, and enables the transceiver to form the received echo signal corresponding to the ultrasonic wave signal.

3. The ultrasonic imaging system of claim 2, wherein the control unit controls the transceiver unit such that the number of the ensembles is reduced by half by halving the number of repetitions of transmission/reception of the ultrasonic wave signal with respect to the scan line for acquisition of the C-mode image.

4. The ultrasonic imaging system of claim 2, wherein the control unit interpolates the ensembles by inserting at least one zero between respective ensembles.

5. The ultrasonic imaging system of claim 2, wherein the control unit interpolates the ensembles by applying a periodic extension to the ensembles and thereby repeating the ensembles one cycle in time domain.

6. The ultrasonic imaging system of claim 2, wherein the control unit interpolates the ensembles by applying a symmetric extension to the ensembles.

7. The ultrasonic imaging system of claim 6, wherein the control unit makes the ensembles symmetric and thereby repeats the symmetric ensembles one cycle in time domain.

8. The ultrasonic imaging system of claim 6, wherein the control unit repeats the symmetric ensembles except for a first occurrence of the symmetric ensembles.

9. The ultrasonic imaging system of claim 2, wherein the control unit interpolates the ensembles by applying an anti-symmetric extension to the ensembles.

10. The ultrasonic imaging system of claim 9, wherein the control unit makes the ensembles anti-symmetric and thereby repeats the anti-symmetric ensembles one cycle in time domain.

11. The ultrasonic imaging system of claim 9, wherein the control unit performs zero insertion once between the ensemble and the anti-symmetric ensemble.

12. The ultrasonic imaging system of claim 1, further comprising:
a signal processor configured to perform low-pass filtering on the interpolated signal and transmit the low-pass-filtered interpolated signal to the image processor.

13. An ultrasonic imaging system, comprising:
a transceiver unit configured to transmit an ultrasonic wave signal for acquisition of a C-mode image to a subject and receive an ultrasonic wave echo signal reflected from the subject;
a storage unit configured to store the received echo signal;
a control unit configured to output an interpolated signal in which zero insertion is performed on one or more ensembles of a scan line for acquisition of the C-mode image identified based on the received echo signal; and
an image processor configured to form the C-mode image based on the interpolated signal and output the C-mode image on a display unit.

14. An ultrasonic imaging system, comprising:
a transceiver unit configured to transmit an ultrasonic wave signal for acquisition of a C-mode image to a subject and receive an ultrasonic wave echo signal reflected from the subject;
a storage unit configured to store the received echo signal;
a control unit configured to repeat one or more ensembles one cycle in time domain by applying a periodic extension to the ensembles of a scan line for acquisition of the C-mode image identified based on the received echo signal and output an interpolated signal; and
an image processor configured to form the C-mode image based on the interpolated signal and output the C-mode image on a display unit.

15. An ultrasonic imaging system, comprising:
a transceiver unit configured to transmit an ultrasonic wave signal for acquisition of a C-mode image to a subject and receive an ultrasonic wave echo signal reflected from the subject;
a storage unit configured to store the received echo signal;
a control unit configured to output an interpolated signal in which a symmetric extension is applied to one or more ensembles of a scan line for acquisition of the C-mode image identified based on the received echo signal;
and
an image processor configured to form the C-mode image based on the interpolated signal and output the C-mode image on a display unit.

16. An ultrasonic imaging system, comprising:
a transceiver unit configured to transmit an ultrasonic wave signal for acquisition of a C-mode image to a subject and receive an ultrasonic wave echo signal reflected from the subject;
a storage unit configured to store the received echo signal;
a control unit configured to output an interpolated signal in which an anti-symmetric extension is applied to one or more ensembles of a scan line for acquisition of the C-mode image identified based on the received echo signal; and
an image processor configured to form the C-mode image based on the interpolated signal and output the C-mode image on a display unit.

17. A color flow imaging method, comprising:
transmitting an ultrasonic wave signal for acquisition of a C-mode image to a subject and receiving an ultrasonic wave echo signal reflected from the subject;
outputting an interpolated signal by interpolating ensembles of at least one scan line for acquisition of the C-mode image identified based on the received echo signal; and
forming the C-mode image based on the interpolated signal and outputting the C-mode image on a display unit.

18. The color flow imaging method of claim 17, wherein the outputting the interpolated signal comprises controlling a transceiver unit such that the ensembles by the number of n are reduced by decreasing the number of repetitions of transmission/reception of the ultrasonic wave signal with respect to the scan line for acquisition of the C-mode image by a predetermined number of times, and enabling the transceiver unit to form the received echo signal corresponding to the ultrasonic wave signal.

19. A color flow imaging method, comprising:
transmitting an ultrasonic wave signal for acquisition of a C-mode image to a subject and receiving an ultrasonic wave echo signal reflected from the subject;
outputting an interpolated signal in which a zero insertion is performed on one or more ensembles of a scan line for acquisition of the C-mode image identified based on the received echo signal; and
forming the C-mode image based on the interpolated signal and outputting the C-mode image on a display unit.

20. A color flow imaging method, comprising:
transmitting an ultrasonic wave signal for acquisition of a C-mode image to a subject and receiving an ultrasonic wave echo signal reflected from the subject;
repeating one or more ensembles one cycle in time domain by applying a periodic extension to the ensembles of a scan line for acquisition of the C-mode image identified based on the received echo signal and outputting an interpolated signal; and
forming the C-mode image based on the interpolated signal and outputting the C-mode image on a display unit.

21. A color flow imaging method, comprising:
transmitting an ultrasonic wave signal for acquisition of a C-mode image to a subject and receiving an ultrasonic wave echo signal reflected from the subject;
outputting an interpolated signal in which a symmetric extension is applied to one or more ensembles of a scan line for acquisition of the C-mode image identified based on the received echo signal; and
forming the C-mode image based on the interpolated signal and outputting the C-mode image on a display unit.

22. A color flow imaging method, comprising:
transmitting an ultrasonic wave signal for acquisition of a C-mode image to a subject and receiving an ultrasonic wave echo signal reflected from the subject;
outputting an interpolated signal in which an anti-symmetric extension is applied to one or more ensembles of a scan line for acquisition of the C-mode image identified based on the received echo signal; and
forming the C-mode image based on the interpolated signal and outputting the C-mode image on a display unit.
